# EUROPEAN PATENT SPECIFICATION

(11) **EP 4 180 117 B1**
(45) Date of publication and mention of the grant of the patent: **18.03.2026**
(21) Application number: 21208521.1
(22) Date of filing: 16.11.2021
(51) Int. Cl.: B01D 61/14, B01D 67/00, B01D 69/02, B01D 71/10, B01D 71/16, B01D 71/20

(54) **MEMBRANE FOR MICROBIOLOGICAL ANALYSIS**
MEMBRAN ZUR MIKROBIOLOGISCHEN ANALYSE
MEMBRANE D'ANALYSE MICROBIOLOGIQUE

(43) Date of publication of application: 17.05.2023
(73) Proprietor: Sartorius Stedim Biotech GmbH, 37079 Göttingen (DE)
(72) Inventor: Ernst, Andrea, 37075 Göttingen (DE); Rüngeling, Elke, 37127 Scheden (DE); Gniewosz, Bastian, 37176 Nörten-Hardenberg (DE); Mazurek-Swidereck, Katharina, 37581 Bad Gandersheim (DE); Wagner, Niels, 37136 Holzerode/Ebergötzen (DE)
(74) Representative: Novagraaf International SA

(56) References cited:
- EP-A2- 0 420 021
- WO-A1-2019/220697
- CN-A- 112 730 823
- DE-A1- 10 102 744
- PRISTOUPIL T I ET AL: "Microimmunoelectrophoresis on nitrocellulose membranes impregnated with Tween 60", CLINICA CHIMICA ACTA, ELSEVIER BV, AMSTERDAM, NL, vol. 14, no. 4, 1 October 1966 (1966-10-01), pages 502 - 504, XP023396828, ISSN: 0009-8981, [retrieved on 19661001], DOI: 10.1016/0009-8981(66)90038-6
- PRISTOUPIL T I ET AL: "Microelectrophoresis of serum proteins on nitrocellulose membranes", JOURNAL OF CHROMATOGRAPHY A, ELSEVIER, AMSTERDAM, NL, vol. 26, 1 January 1967 (1967-01-01), pages 331 - 333, XP026731167, ISSN: 0021-9673, [retrieved on 19670101], DOI: 10.1016/S0021-9673(01)98886-7
- BRENNER KRISTEN P ET AL: "New Screening Test To Determine the Acceptability of 0.45-pLm Membrane Filters for Analysis of Water", APPLIED AND ENVIRONMENTAL MICROBIOLOGY, vol. 56, no. 1, 2 October 1989 (1989-10-02), pages 54 - 64, XP093325841

## Description

The present invention relates to a membrane for microbiological analysis, a production method of a membrane for microbiological analysis, and the use of such membranes for microbiological analysis.

For the accurate detection and quantification of microorganisms in liquid solutions, membrane filters are used in quality control laboratories of beverage manufacturers or water laboratories. These membrane filters are available in various designs and differ in the material used and in their physical and chemical properties. Typically, membrane filters for the detection of microorganisms are made of nitrocellulose (NC), cellulose acetate (CA), polyethersulfone (PESU), polycarbonate (PC) or polyvinylidene fluoride (PVDF), which are available in different nominal pore sizes (0.1 µm - 1.2 µm). The nominal pore size is determined by the characteristic flow times and has to ensure retention of the filtered microorganisms.

In recent years, the influence of membrane filters on the recovery of microorganisms was studied in a large number of publications. Thereby, a significant influence of the chosen material, the pore size as well as the pore size distribution, leachables (i. e. undesirable or hazardous components which are extracted from the membrane during the filtration process), hydrophobic sites and the electrostatic charge of the membrane on the recovery of microorganisms was observed (K. P. Brenner et al., Applied and Environmental Microbiology, 1990, 56 (1), 54-64).

L. Smith et al. (in Applied and Environmental Microbiology, 1993, 59 (1), 344-346) showed that PC membrane filters as well as PESU membrane filters are best suited for *Legionella.* Furthermore, there has been observed a dependence of microorganism growth on the pore size of the membrane. Pores with a nominal pore size of more than 0.8 µm provided the worst recovery results, while a nominal pore size of 0.2 µm provided very good recoveries. However, such low pore sizes lead to a lower filtration performance and make respective membranes unattractive for use in a quality control laboratory for water aiming at high throughput. Until now, PC membrane filters or PESU membrane filters are recommended for filtration of *Legionella.* However, these filters may only be used if the bacteria are subsequently rinsed from the filter and, for example, detection of the bacteria is to be performed by PCR (DIN EN ISO 11731:2017). If the detection of *Legionella* is to be performed by placing the filter directly after filtration on selective agar, the use of filters made of nitrocellulose or cellulose mixed ester is mandatory (DIN EN ISO 11731:2017).

The production of cellulose membranes is well known in the art. For example, DE 10 102 744 A1 discloses surface-treated cellulose membranes which can be produced by phase inversion in an evaporation process. The membrane is treated with brushes or scrapers to remove filter dust occurring during membrane formation, which interferes with the use of the finished membrane for rapid diagnostic tests. EP 3 277 412 A1 discloses the production of diagnostic membranes which can be manufactured from a mixture of membrane-forming polymers and detergents. To fix the detergents in the forming membrane structure, most of the solvents used for membrane production are first removed by drying, before filter dust particles are subsequently removed from the membrane structure and the membrane is completely dried. CN 110146693 A discloses nitrocellulose membranes impregnated with a fluorescent dye and their use in immunochromatographic test strips. During manufacture, phase inversion is controlled by regulating the degree of moisture in the gas phase. CN 108499368 A discloses nitrocellulose membranes reinforced by a paper strip, during the preparation the phase inversion is also controlled by regulating the degree of humidity of the gas phase.

Filters for simple and effective membrane filtration test applications have been available for many years. However, no membrane had been available which does not significantly inhibit the bacterial growth of not only one but various strains, including e.g. *Legionella* and *Alicyclobacillus.*

Thus, the technical problem underlying the present invention is to provide a cellulose membrane which can be used for microbiological analysis in a simple and effective manner and thereby does not significantly inhibit the bacterial growth of not only one but various strains, including e.g. *Legionella* and *Alicyclobacillus.*

The solution to the above technical problem is achieved by the embodiments characterized in the claims.

In particular, the present invention relates to a cellulose membrane for microbiological analysis, wherein the membrane is impregnated with a non-ionic surfactant in an amount of from 100 ng/cm² to 1.0 mg/cm², the membrane has a nominal pore size of from 0.20 µm to 0.80 µm, and the membrane has a cumulative adsorption pore volume of less than 0.010 cm³/g and a specific surface of 4.5 m²/g or less, as measured by the **BET** method.

Herein, the term "microbiological analysis" relates to methods for detecting and quantifying microorganisms in any liquid solutions that are filterable. The liquid solutions are not particularly limited and any liquid solution of interest can be analysed. Examples of respective liquid solutions are samples for quality control, water samples, beverages, microbiological fluid samples, biotechnological process fluids, pharmaceutical solid products after solubilization, and biopharmaceutical process fluids.

The cellulose membrane for microbiological analysis of the present invention does advantageously not inhibit the bacterial growth of various strains. The membrane of the present invention does preferably not show significant wetting defects during or after filtration, due to the specific impregnation with non-ionic surfactants. Wetting defects can occur at hydrophobic points of the filter. These defects become visible after initial wetting of the filter with e.g. water. Unwetted areas are brighter than wetted areas. At these spots, the growth of microorganisms can be inhibited because the nutrients of the agar cannot completely reach the microorganisms via the filter. Due to its good wettability, the membrane of the present invention shows a high recovery ratio with respect to various microorganisms.

Moreover, the membrane of the present invention has a smooth structure on the membrane bridges or membrane web, the membrane bridges or web according to the invention being understood as the solid polymer bulk material surrounding and delimiting the pores. This also advantageously improves the recovery ratio of the membrane of the present invention.

The term "recovery ratio" relates to a ratio of recovered microorganisms after filtration with a membrane filter and growth on culture media relative to the number of microorganisms grown on culture media without previous filtration with a membrane filter (spread plate technique). The recovery ratio can be determined by methods known in the art. For example, there can be employed the method described in ISO 7704:1985. Thereby, the standard ISO11133:2014/AMD1:2018/AMD2:2020 can be taken into account.

In a preferred embodiment, the recovery ratio of a total number of colonies obtained from the membrane filter on a first culture medium to a total number of colonies obtained without usage of the membrane filter on a second culture medium (spread plate on e.g. reference medium or same medium) is from 0.50 to 2.00, more preferably from 0.70 to 1.40, more preferably from 0.80 to 1.20, and most preferably from 0.90 to 1.10. The first and second culture medium may be the same or different and can be appropriately chosen from culture media known in the art. Preferably, the first culture medium is a specific culture medium as designated in a specific standard, such as ISO 9308-1, ISO 7899-2, ISO 11731:2017, ISO 14189, ISO 16266, ISO11133:2020, USP 61, and EP 2.6.12, for use with membrane filters. Examples of specific culture media for use with membrane filters are chromogenic coliform agar (CCA), Slanetz and Bartley medium, Glycine-Vancomycin-Polymoxin B-Cycloheximide agar (GVPC), modified agar after Wadowsky and Yee (MWY), buffered charcoal-yeast extract agar with selective supplements (BCYE+AB), Tryptose-Sulfite-Cycloserine-agar (TSC), and Pseudomonas-CN (pseudomonas selective agar; C: cetrimide, N: nalidixic acid). In case the second culture medium is different from the first culture medium, the second culture medium is preferably a reference culture medium, preferably a non-selective reference culture medium. Examples of reference culture media are tryptone soya agar (TSA), buffered charcoal-yeast extract agar BCYE (Legionallae), and blood agar (Clostrididiae). Duration, temperature and any special conditions, if necessary, of incubation can for example be chosen as mentioned in the standards mentioned above. Counting can for example be carried out in accordance with ISO 8199:2018, USP 61, and EP 2.6.12.

The microorganisms that can be retained by the membrane of the present invention are not limited and can be any microorganisms of interest or isolates found in nature or other environments. For example, the microorganisms can be chosen in accordance with ISO 9308-1 and ISO 7899-2, ISO 11731:2017, ISO 14189, ISO 16266, ISO11133:2020, and USP 61, EP 2.6.12, and other pharmacopeia. Preferably, the microorganism can be chosen from one or more of the group consisting of *Escherichia coli, Enterococcus faecium, Alicyclobacillus acidoterrestris Legionella pneumophila, Legionella anisa,* and *Clostridium perfringens.*

The membrane of the present invention is a cellulose membrane. However, as mentioned above, in principle, also other materials can be used for a membrane for microbiological analysis, such as polyethersulfone (PESU), polycarbonate (PC) or polyvinylidene fluoride (PVDF). Herein, the term "cellulose membrane" means that the membrane contains more than 50 wt% of cellulose derived polymers based on the total weight of the materials constituting the membrane (excluding the compounds which are used for impregnation). Preferably, the membrane of the present invention contains from 60 wt% to 100 wt% of cellulose derived polymers, more preferably from 70 wt% to 100 wt% of cellulose derived polymers, and most preferably from 80 wt% to 100 wt% of cellulose derived polymers. Examples of cellulose derived polymers are cellulose esters, cellulose nitrate and regenerated cellulose. The membrane may independently comprise one or more, preferably one or two, of these membrane-forming polymers. Examples of cellulose esters include cellulose acetate, such as cellulose monoacetate, cellulose diacetate and cellulose triacetate, cellulose propionate, cellulose butyrate and cellulose acetobutyrate. Most preferably, the membrane of the present invention contains a mixture of cellulose acetate and cellulose nitrate as cellulose derived polymers. The weight ratio of cellulose acetate and cellulose nitrate (cellulose acetate:cellulose nitrate) is not particularly limited and may for example be from 100: 1 to 1:1000. Preferably, the ratio is from 50:1 to 1:500, more preferably from 20:1 to 1:200.

The membrane of the present invention is impregnated with a non-ionic surfactant in an amount of from 100 ng/cm² to 1.0 mg/cm². Preferably, the membrane of the present invention is impregnated with a non-ionic surfactant in an amount of from 500 ng/cm² to 500 µg/cm², more preferably from 1.0 µg/cm² to 200 µg/cm², and most preferably from 10 µg/cm² to 100 µg/cm². Impregnation with non-ionic surfactants is beneficial for the growth of microorganisms and ensures homogeneous wetting of the membrane during filtration. Thereby, a high recovery of microorganisms can preferably be achieved.

The non-ionic surfactant used for the impregnation is not particularly limited. For example, as the non-ionic surfactant there can be used one or more non-ionic surfactants selected from the group consisting of Triton X-100 (4-(1,1,3,3-tetramethylbutyl)-phenyl-polyethylene glycol), Tween 80 (polyoxyethylene(x)-sorbitan monooleate (x = 80)), polyoxyethylene(x)-sorbitan monooleate (x = 20, 40, 60, 65), Brij 35 (polyethylene lauryl ether), alcohol alkoxylates (preferably alcohol ethoxylates), and Genapol (polyethylene glycol monoalkyl ether). The non-ionic surfactant is preferably selected from the group consisting of Triton X-100, Tween 80, and Brij 35. Most preferably, the non-ionic surfactant is Triton X-100.

The membrane of the present invention has a nominal pore size of from 0.20 µm to 0.80 µm. The nominal pore size can be determined by measuring the bubble point and a flow rate in accordance with DIN 58355:2011. The categorization can be made in accordance with ASTM F316-03. As a non-limiting example, for a membrane with a nominal pore size of 0.65 µm, the nominal pore size can be determined by measuring the bubble point of 2.1 bar and a flow rate of water of 5 [s/100 mL 0.93 bar 12.5 cm²] for all filter materials. Preferably, the membrane of the present invention has a nominal pore size of from 0.30 µm to 0.75 µm, more preferably from 0.35 µm to 0.70 µm, and most preferably from 0.40 µm to 0.70 µm.

The pore sizes of the microstructure of the membrane of the present invention and their distribution can be determined by BET measurements. The mathematical method according to Barrett, Joyner and Halenda (E. P. Barret et al, The Volume and Area Distribution of Porous Substances, 1951, 73, 373- 380) allows the determination of the distribution of the pore radii from sorption isotherms of BET measurements.

The membrane of the present invention has a cumulative adsorption pore volume of less than 0.010 cm³/g. Preferably, the membrane of the present invention has a cumulative adsorption pore volume of 0.0095 cm³/g or less, more preferably of 0.0090 cm³/g or less, and most preferably of 0.0085 cm³/g or less.

The membrane of the present invention has a specific surface of 4.5 m²/g or less, as measured by the BET method. Most preferably, the membrane of the present invention has a specific surface of 4.0 m²/g or less. Membranes having respective specific surfaces preferably show high recovery of microorganisms.

The thickness of the membrane of the present invention is not particularly limited. For example, the membrane of the present invention has a thickness of from 115 to 145 µm. Preferably, the membrane of the present invention has a thickness of from 115 to 135 µm, more preferably from 120 to 130 µm, and most preferably from 120 to 125 µm.

As the pore size has an influence on the microbial growth of strains, the bubble point of the membrane preferably ranges between 0.3 - 4.0 bar. The membrane of the present invention more preferably has a bubble point of from 1.5 to 3.0 bar, most preferably from 2.0 to 2.5 bar (measured with water, valid for all filter materials).

In a further aspect, the present invention relates to a method of producing a cellulose membrane, comprising the steps of
(a) preparing a feedstock membrane from a cellulose membrane casting solution by phase inversion in an evaporation process,
(b) drying the feedstock membrane, and
(c) impregnating the dried membrane with a non-ionic surfactant by bringing the dried membrane in contact with a surfactant solution having a surfactant concentration of from 0.001 wt% to 1.0 wt% to produce the cellulose membrane,
wherein the step (a) comprises subjecting the applied membrane casting solution to a gas atmosphere containing a non-solvent with respect to membrane polymers, and wherein a relative humidity of the gas atmosphere is from 50% to 100%. The above statements and definitions analogously apply to this aspect of the present invention. The membrane of the present invention can be produced by the method of the present invention.

Evaporation processes for forming membranes by phase inversion are known in the prior art (e.g. from DE 10 102 744 A1) and can be applied in the method of the present invention. The composition of the cellulose membrane casting solution can be appropriately chosen as known in prior art in order to provide a desired membrane composition of the final cellulose membrane. Apart from the membrane-forming polymers, solvents, non-solvents, and various additives can be present in the cellulose membrane casting solution.

Solvents used for the cellulose membrane casting solution are not subject to any particular restrictions, provided they are capable of dissolving the corresponding polymer(s). Preferably, the solvents for the corresponding membrane-forming polymer (or each of the membrane-forming polymers) have a solubility of (in each case) at least 2 wt.%, in particular at least 5 wt.%, more preferably at least 10 wt.%, even more preferably at least 20 wt.%, particularly preferably at least 30 wt.%, at normal conditions (25°C, 1013 hPa). Suitable solvents are known to the skilled person. Suitable solvents for cellulose acetate are, for example, ketones (e.g., acetone), dioxane, amides (e.g., dimethylacetamide (DMAc), N-N-dimethylpropanamide, 2-hydroxy-N,N-dimethylpropanamide) and N-butylpyrrolidone, and mixtures thereof. Suitable solvents for cellulose nitrate are, for example, acetone and ethyl acetate.

The cellulose membrane casting solution may further contain one or more excipients. Suitable excipients include, for example, swelling agents, solubilizers, hydrophilizing agents, pore-forming agents (porogens) and/or non-solvents for the corresponding polymer. Such excipients are known to those skilled in the art and are adapted to the membrane-forming polymer. For example, polyethylene glycol (PEG), in particular PEG 1000 or PEG 2000, glycerol or polyvinylpyrrolidone (PVP) can be used as swelling agents or pore-forming agents. Preferably, the cellulose membrane casting solution consists of the corresponding membrane-forming polymer(s), the solvent, and optionally one or more excipients, preferably pore-forming agents, swelling agents and/or a non-solvent.

Herein, the term "non-solvent" refers to a liquid that is not capable of dissolving the membrane-forming polymer. Preferably, the non-solvent for the membrane-forming polymer (or each of the membrane-forming polymers) has a solubility of (in each case) at most 1 wt.%, particularly preferably at most 0.1 wt.%, under normal conditions.

If a non-solvent (or other precipitant) for the corresponding membrane-forming polymer is present in the cellulose membrane casting solution, the non-solvent (or precipitant) is present at most in a concentration which is insufficient to lead to precipitation of the membrane-forming polymer. Preferably, the cellulose membrane casting solution comprises the solvent for the corresponding membrane-forming polymer, the corresponding membrane-forming polymer, and a non-solvent (mixture).

Suitable non-solvents include, for example, water, glycerol, isopropanol, ethanol, and mixtures thereof. Suitable non-solvents for cellulose acetate are, for example, water, glycerol, isopropanol, ethanol (with decreasing non-solvent properties), and mixtures thereof. Suitable non-solvents for cellulose nitrate are, for example, water and alcohols.

The solids content in the cellulose membrane casting solution is not subject to any particular limitations. Here, the term "solids content" refers to the content of the pure membrane-forming polymer. In a preferred embodiment, the cellulose membrane casting solution has a solids content of from 1 to 30% by weight, in particular from 2 to 20% by weight, even more preferably from 3 to 15% by weight, particularly preferably from 4 to 8% by weight.

As a non-limiting example, the cellulose membrane casting solution may contain 1 to 80 % by weight of solvent, 1 to 70 % by weight of a first non-solvent, 0 to 20 % by weight of a second non-solvent, and 1 to 10 % by weight of membrane-forming polymer.

Processes for applying the cellulose membrane casting solution are known to the skilled person from the prior art and can be used without particular restrictions in the method of the present invention. Such processes are, for example, processes in which a carrier is guided past, for example, a doctor blade system or a slot die from which the corresponding cellulose membrane casting solution emerges. The carrier is not subject to any particular restriction. Any carrier suitable for prior art membrane manufacturing processes can be used. The carrier preferably has a flat surface and is inert to the substances used (e.g. cellulose membrane casting solution and their components). Preferably, the carrier is a moving belt (conveyor belt) or a roll, which enables continuous operation.

The application temperature in step (a) is not subject to any particular limitations. For example, the application temperature may range from 4°C to 40°C. Preferably, the application temperature is from 10°C to 20°C. The application temperature can e.g. be applied to the cellulose membrane casting solution and/or a carrier, when used.

According to the present invention, step (a) comprises subjecting the applied membrane casting solution to a gas (atmosphere) containing a non-solvent with respect to membrane polymers. The above definitions and embodiments with respect to non-solvent apply analogously to the non-solvent contained in the gas, unless otherwise specified. Preferably, step (a) is carried out under defined conditions. The microstructure of the membrane can be influenced by the presence of the non-solvent in the gas (atmosphere). For example, an increasing water content in the gas (atmosphere) can lead to decreasing specific surface of the membrane.

If present, the other constituents of the gas are preferably inert to the substances or equipment used. The remaining components of the gas may be, for example, nitrogen, air, or other gases, preferably nitrogen. Preferably, not only (room) air is used as the gas containing a non-solvent, relative to the polymer in the membrane casting solution. Preferably, the gas does not contain oxygen. For example, the gas containing a non-solvent, based on the polymer in the membrane casting solution, may contain or consist of nitrogen as the carrier gas and a non-solvent (mixture) consisting of 50 to 100% by volume water and 0 to 50% by volume ethanol, preferably 80 to 100% by volume water and 0 to 20% by volume ethanol, more preferably 90 to 100% by volume water and 0 to 10% by volume ethanol, based on the total amount of non-solvent.

The relative humidity (RH) of the gas (atmosphere) is from 50% to 100%, most preferably from 70% to 100%, preferably at a temperature from 30°C to 50°C.

The temperature of the gas (atmosphere) containing the non-solvent with respect to membrane polymers can for example be from 20 to 60°C. Preferably, the temperature is from 30°C to 50°C, most preferably from 35°C to 45°C.

The means that can be used to subject the applied membrane casting solution to the gas atmosphere containing a non-solvent with respect to membrane polymers are not subject to any particular limitations. For example, the applied membrane casting solution may be passed through a chamber (e.g., by means of a carrier) having an appropriate gas atmosphere and in which defined conditions are preferably created. The chamber contains the atmosphere loaded with non-solvent, which is exchanged in such a way that the composition is maintained. In addition, subjection to the gas atmosphere can be achieved by supplying an appropriate gas flow, for example in a channel in which defined conditions are preferably created. Common techniques for gas supply can be used.

In step (a) of the method of the present invention, solvents present in the applied membrane casting solution are preferably removed.

The duration of step (a) is not subject to any particular limitations. For example, the duration may range from 2 min to 120 min. Preferably, the duration is from 2 min to 60 min, more preferably from 5 min to 40 min, most preferably from 10 min to 20 min.

After step (a) and before step (b), the method of the present invention can optionally further comprise a step (a1) of brushing the feedstock membrane obtained after step (a). With such a step (a1) it is possible to remove filter dust. Suitable brushing techniques are for example disclosed in DE 10 102 744 A1.

In step (b) of the method of the present invention, the feedstock membrane is dried. The drying conditions and means are not specifically limited and common drying means and conditions can be used. For example, the drying temperature can be from 15°C to 40°C, preferably from 20°C to 30°C, and the drying time can be from 30 min to 3h, preferably from 60 min to 120 min. The temperature can e.g. be applied to a carrier, when used.

During drying, the gas (atmosphere) can be an inert gas atmosphere or air. Preferably, nitrogen is used as gas during drying.

In step (c) of the method of the present invention, the dried membrane is impregnated with a non-ionic surfactant by bringing the dried membrane in contact with a surfactant solution having a surfactant concentration of from 0.001 wt% to 1.0 wt% to produce the cellulose membrane. Preferably, the surfactant solution has a surfactant concentration of from 0.01 wt% to 0.50 wt%, more preferably from 0.02 wt% to 0.4 wt%, most preferably from 0.05 wt% to 0.2 wt%. The impregnation conditions and means are otherwise not specifically limited and common impregnation means and conditions can be used. For example, the dried membrane can be sprayed with the surfactant solution or immersed in the surfactant solution. After application/immersion, the resulting membrane can be dried by common means to provide the impregnated membrane.

Solvents used for the surfactant solution are not subject to any particular restrictions, provided they are capable of dissolving the corresponding surfactant. Suitable solvents are known to the skilled person. Suitable solvents for non-ionic surfactants are, for example, water, ethanol, methanol, ethyl acetate and mixtures thereof. Preferably, water is used as solvent for the non-ionic surfactant.

In a further aspect, the present invention relates to the use of the cellulose membrane of the present invention or of the cellulose membrane produced by the production method according to the present invention for microbiological analysis. The above statements and definitions analogously apply to this aspect of the present invention.

The cellulose membrane of the present invention can be used for detecting and quantifying microorganisms in liquid solutions.

Preferably, the use of the present invention comprises the steps of retaining one or more microorganisms on the cellulose membrane during filtration of a sample and enumerating the retained microorganisms. The enumerating step can comprise transferring the retained microorganisms on a culture medium, incubating the transferred microorganisms, and evaluating the colonies after incubation. Incubation can be achieved by transferring the membrane filter together with the recovered microorganisms to a solid culture medium or transferring only the recovered microorganisms to the culture medium. Suitable culture media and incubations methods are known to a person skilled in the art. For example, suitable culture media can be chosen in accordance with ISO 9308-1, ISO 7899-2, ISO 11731:2017, ISO 14189, ISO 16266, ISO 11133:2020, and USP 61, EP 2.6.12, and other pharmacopeia. Incubation methods can be chosen in accordance with ISO 8199:2018, ISO 9308-1, ISO 7899-2, ISO 11731:2017, ISO 14189, ISO 16266, ISO 11133:2020, and USP 61, EP 2.6.12, and other pharmacopeia . How to evaluate colonies is known to a person skilled in the art. For example, evaluation can be carried out in accordance with ISO 8199:2018, USP 61, and EP 2.6.12.

The microorganisms retained on the filter can also be removed, e.g. by the method described in ISO 11731:2017 and analyzed further either by conventional incubation in media or by rapid methods, e.g. PCR, flow cytometry, solid phase cytometry, colorimetric methods or chemical methods.
- Fig. 1:: Prior art membrane sample showing non-wetted areas (white) after evaluation with active carbon.
- Figs. 2 to 6:: Evaluation of non-wetted areas on cellulose membranes using active carbon and cellulose membrane filters impregnated by 0.1 wt% of Tween 80 (Figure 2), Triton X-100 (Figure 3), Brij 35 (Figure 4), SDS (Figure 5), and SDBS (Figure 6).
- Fig. 7:: Assembled sample for inverse liquid chromatography (ILC).
- Fig. 8:: Determination of the adsorbed surfactant amount on the cellulose membrane.
- Fig. 9:: AFM image of a membrane bridge or membrane web of a cellulose membrane filter of the present invention.
- Fig. 10:: BET surface area of cellulose membranes with different water contents in the gas phase during membrane formation.
- Fig. 11:: Pore size distribution on membrane bridges or membrane web according to Barrett, Joyner, and Halenda.

The present invention will be further illustrated in the following examples without being limited thereto.

### Example 1: Production of cellulose membrane

In accordance with the inventive Example of DE 10 102 744 A1, a membrane casting solution is made from a polymer blend of commercially available nitrocellulose and cellulose acetate dissolved in a non-solvent, solvent and water. The casting solution is applied to a steel conveyor belt with a thickness of 1400 µm. The coated steel belt passes through a drawing machine with evaporation of the solvent mixture. The desired membrane is formed by phase inversion.

During the evaporation process, the forming membrane is subjected to a gas flow having a temperature of 40°C. The composition of the gas is chosen as desired by mixing nitrogen with different amounts of water vapor (see Example 5). In the subsequent drying step, the membrane is subjected to only a nitrogen gas flow.

The obtained dried membrane is punched into pieces with a diameter of 4.7 cm. These pieces are then placed in the respective impregnation solution for 1.5 minutes and moderately agitated. After 1.5 minutes, the pieces are removed from the impregnation solution and dried at room temperature. Different surfactants have been used for impregnation and the respective membrane filters were evaluated with respect to their properties and the possibility of efficiently recovering various microorganisms after filtration. As non-ionic surfactants Tween 80 (polyoxyethylene(x)-sorbitan monooleate (x = 80)), Triton X100 (4-(1,1,3,3-tetramethylbutyl)-phenyl-polyethylene glycol), and Brij 35 (polyethylene lauryl ether) were used. As anionic surfactants sodium dodecyl sulfate (SDS) and sodium dodecyl benzyl sulfonate (SDBS) were used. The concentrations tested were 0.01 wt%, 0.025 wt%, 0.05 wt%, 0.075 wt%, 0.1 wt%, 0.125 wt%, 0.15 wt%, and 0.2 wt%.

The nominal pore size of the obtained membranes is determined by measuring the bubble point of 2.1 bar and a flow rate of 5 [s/100 mL 0.93 bar 12.5 cm²] in accordance with DIN 58355:2011. For all membranes a nominal pore size of 0.65 µm has been determined.

### Example 2: Evaluation of wettability

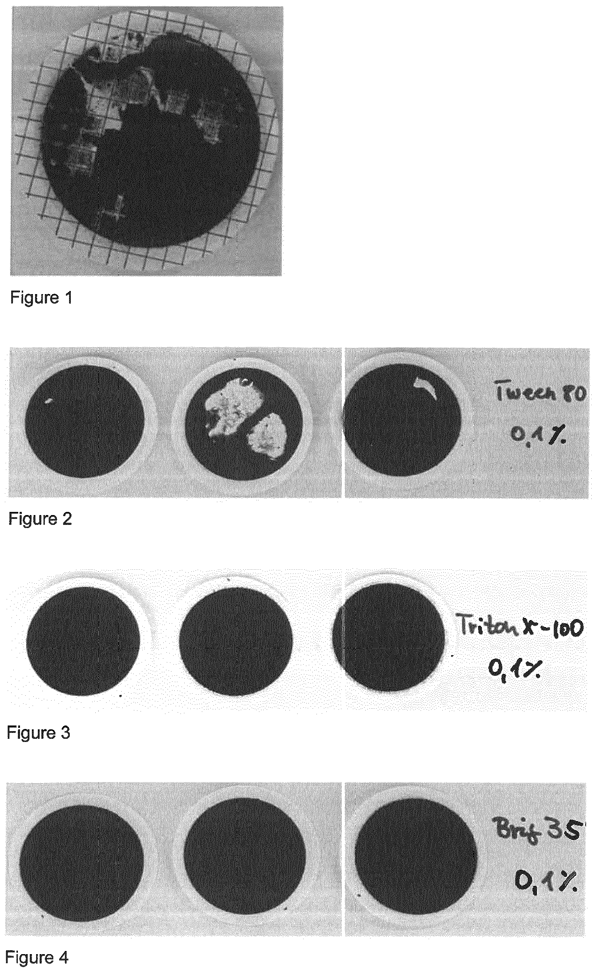

The wetting ability of membrane filters obtained in Example 1, which were impregnated with 0.1 wt% of each of Tween 80, Triton X100, Brij 35, SDS, and SDBS, was evaluated after filtration with activated carbon. For this purpose, the pieces with a diameter of 4.7 cm were placed with the application side of the membrane pointing upwards on a frit of a filtration unit before cups are attached thereon. All valves of the filtration bar are closed. 0.2 g of activated carbon is suspended in 1 L of RO water and 100 mL of filtration volume is measured after vigorous stirring and filled into the filtration cups. The pump is then switched on and the valves are opened. After filtration, the black filter pieces are placed on a white sheet of paper and photographed. White spots indicate an inhomogeneous wetting pattern (Figure 1). With the exception of Tween 80, for which white wetting spots were visible (Figure 2), all other surfactants showed a homogeneous black wetting pattem (Figure 3 to 6).

### Example 3: Evaluation of microbial growth

The membrane filters obtained in Example 1 were evaluated with regard to microbial growth. E. *coli, E. faecium, L. anisa and A. acidoterrestris* were selected as critical test bacteria. The procedure is described in ISO 8199:2018 (general description), ISO 9308-1 (*E. coli*), ISO 7899-2 (E. *faecium),* and ISO 11731:2017 *(L. anisa)* and USP 61, EP 2.6.12, and other pharmacopeia.

The filter samples were exposed to the four microorganisms (Table 1) for the evaluation of microbial recovery. Spatulated agar plates without filters served as reference. The tests were performed in quintuplicate.

**Table 1**

| strain | WDCM No. | ATCC | DSMZ | media |
|---|---|---|---|---|
| *A. acidoterrestris* | | 49025 | 3922 | BAT |
| *E. faecium* | 00177 | 6057 | 2146 | Slanetz & Bartley |
| *E. coli* | 00012 | 8739 | 1576 | CCA |
| *L. anisa* | 00106 | 35292 | 17627 | GVPC |

The titer of the bacterial suspension is adjusted so that there are 50 -150 cfu on the agar control. 100 µl of the bacterial suspension is spatulated out onto agar plates. Using membrane filtration method, 100 µl of the bacterial suspension is added to 10 ml of buffer (phosphate buffer; sterile tap water for *L. anisa*) and then filtered over the filter samples. Immediately after filtration, the samples are transferred to the designated agar media and incubated as follows:

**Table 2**

| strain | incubation conditions |
|---|---|
| *A. acidoterrestris* | 44°C for 48±2h |
| *E. faecium* | 37°C for 48±2h |
| *E. coli* | 37°C for 24±2h |
| *L. anisa* | 37°C for minimum 5d |

After incubation, the grown colonies are counted and the recovery ratio on the filters is calculated. The respective results are summarized in Table 3. In addition to the colony counts, the growth pattern has been assessed and conforms with respective standards ISO 8199:2018 (general description), ISO 9308-1 (*E. coli*), ISO 7899-2 (E. *faecium*), and ISO 11731:2017 (*L. anisa*) and USP 61, EP 2.6.12, and other pharmacopeia.

**Table 3**

| | *E. coli* WDCM 00012 | *E. faecium* WDCM 00177 | *A. acidoterrestris* ATCC 49025 | *L. anisa* WDCM 00106 |
|---|---|---|---|---|
| Tween80 0.01wt% | 98% | 105% | 164% | 129% |
| Tween80 0.025wt% | 93% | 106% | 166% | 113% |
| Tween80 0.05wt% | 79% | 103% | 170% | 97% |
| Tween80 0.075wt% | 94% | 115% | 127% | 98% |
| Tween80 0.1wt% | 86% | 106% | 168% | 105% |
| Tween80 0.125wt% | 102% | 108% | 169% | 78% |
| Tween80 0.15wt% | 98% | 80% | 138% | 87% |
| Tween80 0.2wt% | 77% | 104% | 136% | 92% |
| Triton X-100 0.01wt% | 91% | 112% | 102% | 101% |
| Triton X-100 0.025wt% | 85% | 99% | 118% | 113% |
| Triton X-100 0.05wt% | 82% | 100% | 139% | 94% |
| Triton X-100 0.075wt% | 96% | 91% | 143% | 113% |
| Triton X-100 0.1wt% | 98% | 105% | 150% | 101% |
| Triton X-100 0.125wt% | 101% | 104% | 148% | 82% |
| Triton X-100 0.15wt% | 69% | 88% | 175% | 90% |
| Triton X-100 0.2wt% | 83% | 93% | 186% | 80% |
| Brij 35 0.01 wt% | 76% | 95% | 189% | 102% |
| Brij 35 0.025 wt% | 80% | 89% | 151% | 98% |
| Brij 35 0.05 wt% | 70% | 85% | 191% | 85% |
| Brij 35 0.075 wt% | 86% | 103% | 136% | 84% |
| Brij 35 0.1 wt% | 71% | 93% | 141% | 84% |
| Brij 35 0.125 wt% | 75% | 90% | 125% | 96% |
| Brij 35 0.15 wt% | 84% | 103% | 127% | 87% |
| Brij 35 0.2 wt% | 88% | 106% | 118% | 74% |
| SDBS 0.01 wt% | 86% | 85% | 82% | 153% |
| SDBS 0.025 wt% | 98% | 88% | 54% | 147% |
| SDBS 0.05 wt% | 88% | 88% | 30% | 151% |
| SDBS 0.075 wt% | 72% | 91% | 32% | 127% |
| SDBS 0.1 wt% | 89% | 89% | 30% | 114% |
| SDBS 0.125 wt% | 92% | 98% | 50% | 113% |
| SDBS 0.15 wt% | 84% | 91% | 118% | 61% |
| SDBS 0.2 wt% | 82% | 96% | 150% | 26% |
| SDS 0.01 wt% | 88% | 114% | 156% | 137% |
| SDS 0.025 wt% | 82% | 111% | 122% | 140% |
| SDS 0.05 wt% | 85% | 107% | 126% | 129% |
| SDS 0.075 wt% | 85% | 108% | 109% | 146% |
| SDS 0.1 wt% | 82% | 103% | 72% | 137% |
| SDS 0.125 wt% | 100% | 108% | 72% | 115% |
| SDS 0.15 wt% | 87% | 106% | 0% | 124% |
| SDS 0.2 wt% | 80% | 108% | 0% | 135% |

As an example a recovery rate of 72% as outlined in Table 3 corresponds to a value of 0.72 according to ISO 7704 (revision under development; preview available under: www.iso.org) in line with Section 10.2 and Annex C, Section C, C.3 of ISO 7704.

The evaluation of the recovery of the colonies compared to the recovery of the microorganism without filter shows that *E. coli* and *E*. *faecium* do not show a significant dependence on the surfactant used. The situation is different, however, for A. *acidoterrestris.* In membranes impregnated with SDS, low microbial growth is consistently seen. A decrease in microbial growth is even observed for membrane filters impregnated with higher concentrations of SDBS (from 0.1 wt%). When the non-ionic surfactants are used, the recovery is consistently above 100%. A similar effect is seen with *L. anisa.* Here, a sharp decrease in colony forming units (cfu) is observed with the SDBS impregnated membranes especially at higher surfactant concentrations (0.1 wt% - 0.2 wt%). For the surfactant concentrations between 0.01 wt% and 0.075 wt%, the recoveries are more in the medium range. Impregnations with Triton X-100 give the best results.

### Example 4: Evaluation of amount of adsorbed surfactants

The amount of adsorbed surfactants can be determined by inverse liquid chromatography (ILC) as described in the following.

### Devices and materials

Inverse liquid chromatography (ILC) system from Knauer (configuration from product numbers: EZC00, DYBQEKEA, DYGAGAGA, EDB01, EPH34)
Non-impregnated cellulose membrane
Filter table MA15 from Sartorius Stedim Plastics GmbH
Cover MA15 from Sartorius Stedim Plastics GmbH
Stainless steel housing upper part (self-made)
Stainless steel housing lower part (self-made)

### Sample preparation

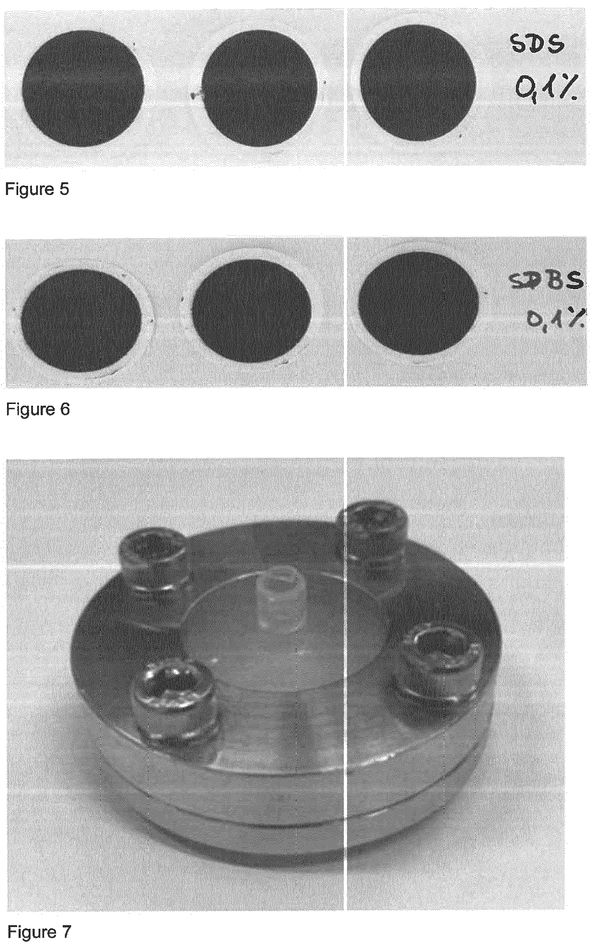

Three round pieces with a diameter of 30 mm are punched from the non-impregnated cellulose membrane of Example 1. The membrane is placed on the MA15 filter table with the carrier side or application side facing down. The MA15 cover is then placed on the MA15 filter table and the Minisart that has just been assembled is placed in the lower part of the stainless steel housing with the filter table pointing downwards. The upper part of the stainless steel housing is placed on the lower part and fixed with four screws (Figure 7). This process is carried out a total of three times. In addition, this process is carried out a further six times, but without the cellulose membrane (referred to as blanks).

### Preparation of the ILC

ILC is carried out by applying the software "PurityChrom" Version 5.9. The ILC is equipped with six sample tubes with which a total of six different sample solutions can be subjected to suction. In addition, the ILC is equipped with 15 membrane valves and a bypass, which makes it possible to test 15 different samples one after the other.

Six blanks and three housings with the cellulose membrane are now attached to the sample table in the following step. Three connectors are installed as standard and are measured as well.

### Description of the measuring method

Each connector, each sample and each blank are measured with the same measuring method, which is described in the following. All steps are stored in the measuring method and are executed automatically. The system first switches to the sample hose S1, which ensures that deionized water can be sucked in, and then switches to the membrane valve to be measured with the sample, blank or connector. The flow of the pump is set to a flow rate of 0.2 ml and a UV detector detects the intensity at a wavelength of 220 nm. This is followed by an autozero of the UV detector and the conductivity detector. Starting from here, a chromatogram is recorded with the wavelength of 220 nm and the conductivity. A total of 10 ml of deionized water is pumped through the respective membrane valve, which corresponds to a duration of 50 minutes. Subsequently, the system switches to the sample hose S2, which conveys 15 ml of a specific surfactant solution through the system or via the membrane valves, respectively. Once this step is completed, the system switches back to sample hose S1 and a further 25 ml of deionized water are conveyed. Then, the recording of the chromatogram is stopped and the measurement for one membrane valve is complete. One measurement takes a total of 250 minutes.

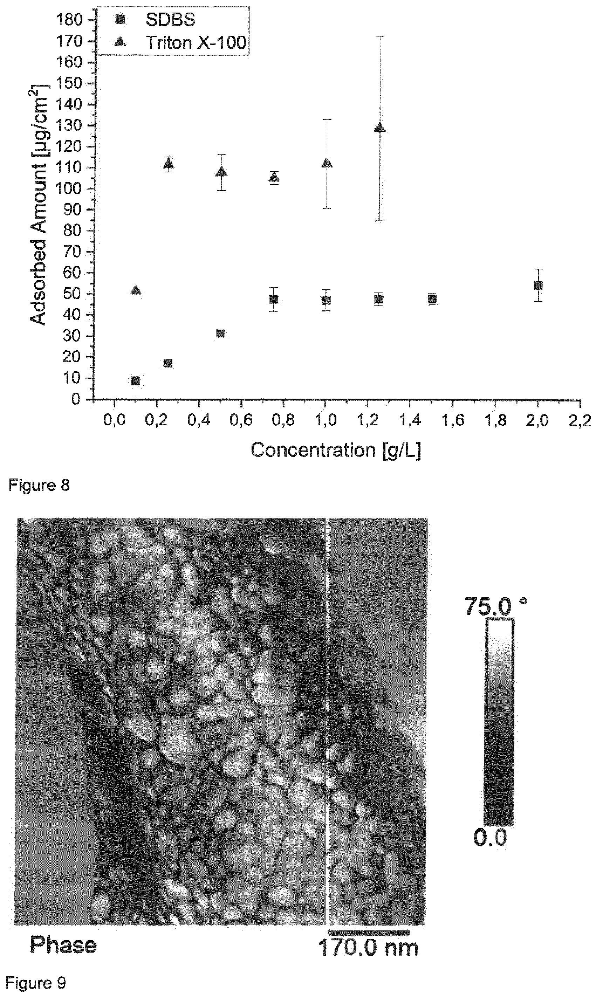

Accordingly, in this method, an aqueous surfactant solution is conveyed through the membrane at a constant volume flow rate. A certain amount of surfactant adsorbs on the membrane depending on the surfactant concentration (Figure 8).

When comparing the adsorbed amounts, it is noticeable that the non-ionic surfactant Triton X-100 adsorbs in significantly higher amounts on the membrane. In particular, at the lower concentration of 0.1 g/L, the adsorbed amount of Triton X-100 (i.e. 51.5 µg/cm² ± 0.6 µg/cm²) is greater than for the anionic surfactant SDBS (8.73 µg/cm² ± 0.97 µg/cm²). At a concentration of 1.0 g/L, the adsorbed amount of Triton X-100 is two times higher compared to SDBS.

### Example 5: Evaluation of the influence of the cellulose membrane structure on microbial growth

The structure of the membrane bridges or membrane web is analyzed by using atomic force microscopy (AFM). Thereby, the cellulose membrane filters show a porous microstructure on the membrane bridges or membrane web (Figure 9). In addition, images taken with confocal microscopy (CLSM) using *E*. *coli as* an example showed that the microorganisms penetrate approx. 5 µm into the membrane and form colonies from there.

For studying the influence of the microstructure on the growth of the microorganisms, various cellulose membrane structures were produced by varying water vapor contents in the gas atmosphere during membrane formation (cf. Example 1). In particular, 0%, 25%, 50%, 75% and 100% of water (in terms of RH) were added to the nitrogen volume flow. All cellulose membranes were prepared with a nominal pore size of 0.45 µm and subsequently analyzed by BET measurements using a Gemini BET test station (micromeritics) with Gemini 2390T with Dewar, Vac Prep, 2 vacuum pumps, PC and monitor and using Gemini VII software, version 5.01. For reproduceable results 0.3 g cellulose membrane is weight into the vial and dried thoroughly for at least 2 h under vacuum. As comparative membranes, there were used two different prior art membranes, which are referred to as references 1 (nitrocellulose membrane, nominal pore size 0.45 µm, thickness > 100 µm, wetting time < 5 sec, typical flow rate 100 mL/min cm² bar) and reference 2 (nitrocellulose membrane, nominal pore size 0.45 µm, thickness 115 - 180 µm, flow through time 25 - 50 s/500 mL @47mm at 27.5 ± 0.5 inches Hg).

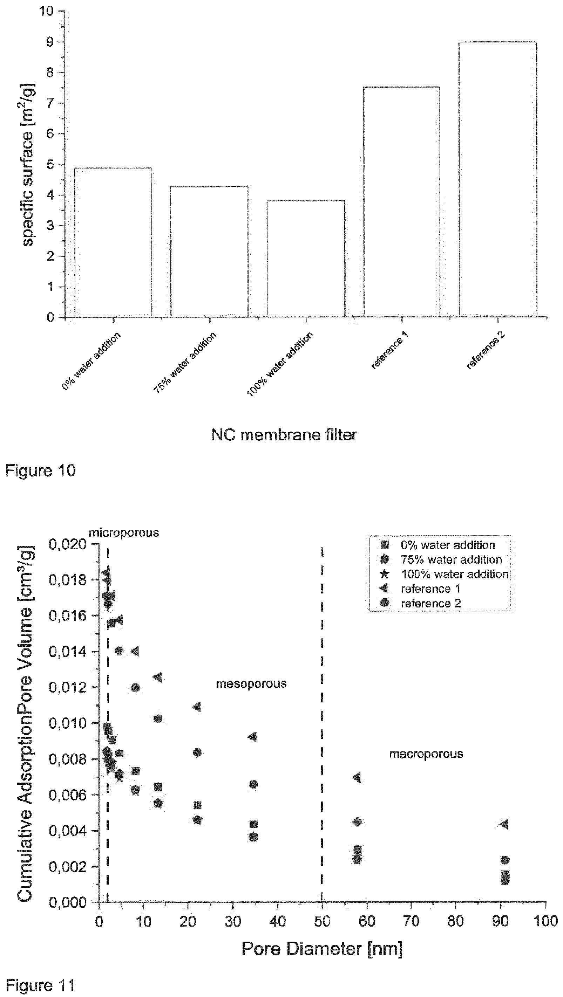

Evaluation of the BET surface area shows that it tends to decrease with higher water content in the gas atmosphere (Figure 10). This trend correlates with the recovery of *L. anisa* observed for the respective membranes. The lower the specific surface of the membrane, the higher the recovery of the microorganisms. In particular, references 1 and 2 having a rather high specific surface show no (reference 1) or minimal (reference 2) growth *of L. anisa* on the membrane (Table 4).

**Table 4**

| | 0% water | 25% water | 50% water | 75% water | 100% water | reference 1 | reference 2 |
|---|---|---|---|---|---|---|---|
| recovery ratio [%] for *L. anisa* | 75 | 76 | 90 | 98 | 110 | 0 | 34 |

The pore sizes of the microstructure and their distribution can be determined by BET measurements. The mathematical method according to Barrett, Joyner and Halenda (E. P. Barret et al, The Volume and Area Distribution of Porous Substances, 1951, 73, 373-380) allows the determination of the distribution of pore radii from sorption isotherms of BET measurements (Figure 11).

When comparing the curves in Figure 11 without water enrichment (squares) and with 75% (pentagons) and 100% (stars) water enrichment in the gas flow during the membrane formation phase, it is noticeable that without water enrichment an overall higher number of pores can be found on the membrane bridges or membrane web. Especially in the microporous (≤ 2 nm) and mesoporous (2-50 nm) range, the difference is clearly visible. The lower total number of pores promotes the recovery of *L. anisa* on the cellulose membrane filter. This is also confirmed by the analysis of the BJH distribution of references 1 and 2. Reference 2 still shows a recovery rate of 34%, while no colonies of *L. anisa* are recovered on reference 1 having the highest porosity.

It has been shown that various microorganisms can be reliably recovered after filtration with the cellulose membrane of the present invention. The selection of a non-ionic surfactant for impregnating the membrane can greatly improve the wettability of the membrane and can improve the recovery of the microorganisms. Furthermore, the microstructure of the membrane supports the growth of *L. anisa.* The smaller the pores on the membrane bridges or membrane web, the better *L. anisa* grows on the membrane. This has been shown by the BET and BJH analyses of the different cellulose membrane filters. The combination of the two features leads to a significant improvement of cellulose membrane filters for use in the detection and quantification of microorganisms.

## Claims

1. A cellulose membrane for microbiological analysis, wherein the membrane is impregnated with a non-ionic surfactant in an amount of from 100 ng/cm² to 1.0 mg/cm²,
the membrane has a nominal pore size of from 0.20 µm to 0.80 µm, and
the membrane has a cumulative adsorption pore volume of less than 0.010 cm³/g, wherein the nominal pore size is determined by measuring the bubble point and a flow rate in accordance with DIN 58355:2011 and categorizing in accordance with ASTM F316-03,
wherein the cumulative adsorption pore volume is determined as described herein, and
wherein the membrane has a specific surface of 4.5 m²/g or less, as measured by the BET method.

2. The cellulose membrane according to claim 1, wherein the non-ionic surfactant is selected from the group consisting of 4-(1,1,3,3-tetramethylbutyl)-phenyl-polyethylene glycol, polyoxyethylene(x)-sorbitan monooleate (x = 80), polyoxyethylene(x)-sorbitan monooleate (x = 20, 40, 60, 65), polyethylene lauryl ether, alcohol alkoxylates, preferably alcohol ethoxylates, and polyethylene glycol monoalkyl ether.

3. The cellulose membrane according to claim 1 or 2, wherein the membrane has a nominal pore size of from 0.40 µm to 0.70 µm.

4. The cellulose membrane according to any one of claims 1 to 3, wherein the membrane has a thickness of from 115 to 145 µm.

5. A method of producing a cellulose membrane according to any one of claims 1 to 4, comprising the steps of
(a) preparing a feedstock membrane from a cellulose membrane casting solution by phase inversion in an evaporation process,
(b) drying the feedstock membrane, and
(c) impregnating the dried membrane with a non-ionic surfactant by bringing the dried membrane in contact with a surfactant solution having a surfactant concentration of from 0.001 wt% to 1.0 wt% to produce the cellulose membrane,
wherein the step (a) comprises subjecting the applied membrane casting solution to a gas atmosphere containing a non-solvent with respect to membrane polymers, and
wherein the non-solvent is water and a relative humidity of the gas atmosphere is from 50% to 100%.

6. The method according to claim 5, wherein the gas atmosphere does not contain oxygen.

7. The method according to claim 5 or 6, wherein the non-ionic surfactant is selected from the group consisting of 4-(1,1,3,3-tetramethylbutyl)-phenyl-polyethylene glycol, polyoxyethylene(x)-sorbitan monooleate (x = 80), polyoxyethylene(x)-sorbitan monooleate (x = 20, 40, 60, 65), polyethylene lauryl ether, alcohol alkoxylates, preferably alcohol ethoxylates, and polyethylene glycol monoalkyl ether.

8. The method according to any one of claims 5 to 7, wherein the method further comprises, after step (a) and before step (b), a step (a1) of brushing the feedstock membrane obtained after step (a).

9. Use of the cellulose membrane according to any one of claims 1 to 4 or the cellulose membrane obtained by the method according to any one of claims 5 to 8 for microbiological analysis.

10. The use according to claim 9, comprising the steps of retaining one or more microorganisms during filtration of a sample and enumerating the retained microorganisms.

11. The use according to claim 10, wherein the one or more microorganisms are selected from the group consisting of *Escherichia coli, Enterococcus faecium, Alicyclobacillus acidoterrestris,* and *Legionella anisa.*

12. The use according to any one of claims 9 to 11, wherein the recovery ratio of a total number of colonies obtained from the membrane filter on a first culture medium to a total number of colonies obtained without usage of the membrane filter on a second culture medium is from 0.50 to 2.00.

## Patentansprüche

1. Cellulosemembran für mikrobiologische Analyse, wobei die Membran mit einem nichtionischen Tensid in einer Menge von 100 ng/cm² bis 1,0 mg/cm² imprägniert ist,
die Membran eine nominelle Porengröße von 0,20 µm bis 0,80 µm aufweist, und
die Membran ein kumulatives Adsorptionsporenvolumen von weniger als 0,010 cm³/g aufweist,
wobei die nominelle Porengröße durch Messung des Blasenpunkts und einer Durchflussrate gemäß DIN 58355:2011 bestimmt und gemäß ASTM F316-03 kategorisiert wird,
wobei das kumulative Adsorptionsporenvolumen wie hierin beschrieben bestimmt wird,
und
wobei die Membran eine spezifische Oberfläche von 4,5 m²/g oder weniger, gemessen nach der BET-Methode, aufweist.

2. Cellulosemembran nach Anspruch 1, wobei das nichtionische Tensid aus der Gruppe bestehend aus 4-(1,1,3,3-Tetramethylbutyl)-phenyl-polyethylenglykol, Polyoxyethylen(x)-sorbitan-monooleat (x = 80), Polyoxyethylen(x)-sorbitan-monooleat (x = 20, 40, 60, 65), Polyethylenlaurylether, Alkoholalkoxylaten, vorzugsweise Alkoholethoxylaten, und Polyethylenglykolmonoalkylether ausgewählt ist.

3. Cellulosemembran nach Anspruch 1 oder 2, wobei die Membran eine nominelle Porengröße von 0,40 µm bis 0,70 µm aufweist.

4. Cellulosemembran nach einem der Ansprüche 1 bis 3, wobei die Membran eine Dicke von 115 bis 145 µm aufweist.

5. Verfahren zur Herstellung einer Cellulosemembran nach einem der Ansprüche 1 bis 4, umfassend die Schritte
(a) Herstellen einer Rohmembran aus einer Cellulosemembran-Gießlösung durch Phaseninversion in einem Verdampfungsprozess,
(b) Trocknen der Rohmembran, und
(c) Imprägnieren der getrockneten Membran mit einem nichtionischen Tensid durch Inkontaktbringen der getrockneten Membran mit einer Tensidlösung mit einer Tensidkonzentration von 0,001 Gew.-% bis 1,0 Gew.-% zur Herstellung der Cellulosemembran,
wobei der Schritt (a) das Aussetzen der aufgebrachten Membran-Gießlösung einer Gasatmosphäre umfasst, die ein Nichtlösungsmittel in Bezug auf membranbildende Polymere enthält,
und
wobei das Nichtlösungsmittel Wasser ist und eine relative Feuchtigkeit der Gasatmosphäre 50 % bis 100 % beträgt.

6. Verfahren nach Anspruch 5, wobei die Gasatmosphäre keinen Sauerstoff enthält.

7. Verfahren nach Anspruch 5 oder 6, wobei das nichtionische Tensid aus der Gruppe bestehend aus 4-(1,1,3,3-Tetramethylbutyl)-phenyl-polyethylenglykol, Polyoxyethylen(x)-sorbitan-monooleat (x = 80), Polyoxyethylen(x)-sorbitan-monooleat (x = 20, 40, 60, 65), Polyethylenlaurylether, Alkoholalkoxylaten, vorzugsweise Alkoholethoxylaten, und Polyethylenglykolmonoalkylether ausgewählt ist.

8. Verfahren nach einem der Ansprüche 5 bis 7, wobei das Verfahren ferner nach Schritt (a) und vor Schritt (b) einen Schritt (a1) des Bürstens der nach Schritt (a) erhaltenen Rohmembran umfasst.

9. Verwendung der Cellulosemembran nach einem der Ansprüche 1 bis 4 oder der durch das Verfahren nach einem der Ansprüche 5 bis 8 erhaltenen Cellulosemembran für mikrobiologische Analyse.

10. Verwendung nach Anspruch 9, umfassend die Schritte des Zurückhaltens eines oder mehrerer Mikroorganismen während der Filtration einer Probe und des Zählens der zurückgehaltenen Mikroorganismen.

11. Verwendung nach Anspruch 10, wobei der eine oder die mehreren Mikroorganismen aus der Gruppe bestehend aus *Escherichia coli, Enterococcus faecium, Alicyclobacillus acidoterrestris* und *Legionella anisa* ausgewählt sind.

12. Verwendung nach einem der Ansprüche 9 bis 11, wobei das Wiederfindungsverhältnis einer Gesamtzahl von Kolonien, die aus dem Membranfilter auf einem ersten Kulturmedium erhalten wurden, zu einer Gesamtzahl von Kolonien, die ohne Verwendung des Membranfilters auf einem zweiten Kulturmedium erhalten wurden, 0,50 bis 2,00 beträgt.

## Revendications

1. Membrane cellulosique pour analyse microbiologique, dans laquelle la membrane est imprégnée d'un tensioactif non ionique en une quantité de 100 ng/cm² à 1,0 mg/cm²,
la membrane a une taille de pore nominale de 0,20 µm à 0,80 µm, et
la membrane a un volume poreux d'adsorption cumulatif inférieur à 0,010 cm³/g,
dans laquelle la taille de pore nominale est déterminée par mesure du point de bulle et d'un débit conformément à la norme DIN 58355:2011 et catégorisation conformément à la norme ASTM F316-03,
dans laquelle le volume poreux d'adsorption cumulatif est déterminé comme décrit dans la présente,
et
dans laquelle la membrane a une surface spécifique de 4,5 m²/g ou moins, telle que mesurée par la méthode BET.

2. Membrane cellulosique selon la revendication 1, dans laquelle le tensioactif non ionique est sélectionné dans le groupe constitué par le 4-(1,1,3,3-tétraméthylbutyl)-phényl-polyéthylène glycol, le monoléate de polyoxyéthylène(x)-sorbitan (x = 80), le monoléate de polyoxyéthylène(x)-sorbitan (x = 20, 40, 60, 65), l'éther laurylique de polyéthylène, les alkoxylates d'alcool, de préférence les éthoxylates d'alcool, et l'éther monoalkylique de polyéthylène glycol.

3. Membrane cellulosique selon la revendication 1 ou 2, dans laquelle la membrane a une taille de pore nominale de 0,40 µm à 0,70 µm.

4. Membrane cellulosique selon l'une quelconque des revendications 1 à 3, dans laquelle la membrane a une épaisseur de 115 à 145 µm.

5. Procédé de production d'une membrane cellulosique selon l'une quelconque des revendications 1 à 4, comprenant les étapes de
(a) préparation d'une membrane brute à partir d'une solution de coulée de membrane cellulosique par inversion de phase dans un procédé d'évaporation,
(b) séchage de la membrane brute, et
(c) imprégnation de la membrane séchée avec un tensioactif non ionique en mettant la membrane séchée en contact avec une solution de tensioactif ayant une concentration en tensioactif de 0,001 % en poids à 1,0 % en poids pour produire la membrane cellulosique,
dans lequel l'étape (a) comprend la soumission de la solution de coulée de membrane appliquée à une atmosphère gazeuse contenant un non-solvant par rapport aux polymères formant la membrane,
et
dans lequel le non-solvant est l'eau et une humidité relative de l'atmosphère gazeuse est de 50 % à 100 %.

6. Procédé selon la revendication 5, dans lequel l'atmosphère gazeuse ne contient pas d'oxygène.

7. Procédé selon la revendication 5 ou 6, dans lequel le tensioactif non ionique est sélectionné dans le groupe constitué par le 4-(1,1,3,3-tétraméthylbutyl)-phényl-polyéthylène glycol, le monoléate de polyoxyéthylène(x)-sorbitan (x = 80), le monoléate de polyoxyéthylène(x)-sorbitan (x = 20, 40, 60, 65), l'éther laurylique de polyéthylène, les alkoxylates d'alcool, de préférence les éthoxylates d'alcool, et l'éther monoalkylique de polyéthylène glycol.

8. Procédé selon l'une quelconque des revendications 5 à 7, dans lequel le procédé comprend en outre, après l'étape (a) et avant l'étape (b), une étape (a1) de brossage de la membrane brute obtenue après l'étape (a).

9. Utilisation de la membrane cellulosique selon l'une quelconque des revendications 1 à 4 ou de la membrane cellulosique obtenue par le procédé selon l'une quelconque des revendications 5 à 8 pour l'analyse microbiologique.

10. Utilisation selon la revendication 9, comprenant les étapes de rétention d'un ou plusieurs micro-organismes pendant la filtration d'un échantillon et de dénombrement des micro-organismes retenus.

11. Utilisation selon la revendication 10, dans laquelle le ou les micro-organismes sont sélectionnés dans le groupe constitué par *Escherichia coli, Enterococcus faecium, Alicyclobacillus acidoterrestris* et *Legionella anisa.*

12. Utilisation selon l'une quelconque des revendications 9 à 11, dans laquelle le taux de récupération d'un nombre total de colonies obtenues à partir du filtre membranaire sur un premier milieu de culture par rapport à un nombre total de colonies obtenues sans utilisation du filtre membranaire sur un second milieu de culture est de 0,50 à 2,00.
